# DEMANDE DE BREVET EUROPEEN

(11) **EP 0 542 629 A1**
(43) Date de publication de la demande: **19.05.1993**
(21) Numéro de dépôt: 92403055.4
(22) Date de dépôt: 12.11.1992
(51) Int. Cl.: C12N 15/55, C12N 9/20, A61K 37/54, C12N 11/00

(54) **Acides nucléiques codant pour la lipase gastrique de lapin et dérivés polypeptidiques, leur utilisation pour la production de ces polypeptides, et compositions pharmaceutiques à base de ces derniers**

(30) Priorité: 13.11.1991 FR 9113948
(71) Demandeur: INSTITUT DE RECHERCHE JOUVEINAL (I.R.J), F-94265 Fresnes Cedex (FR)
(72) Inventeur: Benicourt, Claude, F-78800 Houilles (FR); Blanchard, Claire, F-91350 Grigny (FR); Junien, Jean-Louis, F-92310 Sevres (FR)
(74) Mandataire: Grosset-Fournier, Chantal Catherine

(57) **Abrégé**

La présente invention concerne la lipase gastrique de lapin telle qu'obtenue par voie du génie génétique ainsi que la séquence de nucléotides codant pour cette LGL recombinante.

Elle vise également l'utilisation de cette LGL recombinante pour l'obtention de compositions pharmaceutiques destinées, notamment, au traitement de pathologies liées à l'insuffisance, voire à l'absence de sécrétion de lipase gastrique dans l'organisme d'un individu.

## Description

La présente invention concerne des acides nucléiques codant pour la lipase gastrique de lapin (LGL), et autres dérivés polypeptidiques de cette dernière possédant une activité lipasique, ainsi que leurs utilisations, notamment pour la production de ces polypeptides. L'invention a également pour objet les polypeptides codés par ces acides nucléiques, et l'utilisation de ces polypeptides dans des compositions pharmaceutiques.

La LGL est une glycoprotéine de 376 acides aminés (AA) d'un poids moléculaire de 50 kilodaltons (kD) synthétisée sous forme d'un précurseur contenant un peptide signal à l'extrémité aminoterminale (NH₂-terminale) et sécrétée par les cellules médianes de la muqueuse fundique de l'estomac de lapin (Moreau H. et al., Biochem. Biophys. Act. 960 (1988) 286-293). Cette enzyme appartient à une famille de lipases dites "préduodénales" dont certains membres ont déjà été purifiés et parfois même clonés (Docherty A.J.P. et al., Nucl. Ac. Res. 13 (1985) 1891-1903; Bodmer et al., Biochem. Biophys. Act. 909 (1987) 237-244).

Pendant longtemps, on a tenu pour acquis que l'hydrolyse des lipides alimentaires s'effectuait au niveau de l'intestin grêle grâce à l'action d'enzymes produites par le pancréas (Bernard C., C.R. Acad. Sci. 28 (1849) 249-253).

Des observations laissaient cependant penser que l'hydrolyse de triglycérides pouvait avoir lieu dans l'estomac par le biais d'enzymes préduodénales (Volhard, F., Z. Klin. Med. 42 (1901) 414-429; Shonheyder, F. and Volquartz, K. Acta Physiol. Scand. 9 (1945) 57-67). Ces enzymes, et en particulier la lipase gastrique de lapin, ont des propriétés enzymatiques et physico-chimiques qui les différencient des lipases pancréatiques de mammifères. Ces différences entre lipases gastrique et pancréatique concernent essentiellement les points suivants: poids moléculaire, composition en acides aminés, résistance à la pepsine, spécificité de substrat, pH optimum d'action, et stabilité en milieu acide.

De plus, in vitro, dans certaines conditions, on peut mettre en évidence une synergie d'action entre les lipases gastrique et pancréatique sur l'hydrolyse de triglycérides à chaînes longues (Gargouri, Y. et al., Biochem. Biophys. Act. 1006 (1989) 255-271).

On connaît plusieurs situations pathologiques (mucoviscidose, insuffisance pancréatique exocrine) où les patients sont totalement ou partiellement dépourvus de sécrétion pancréatique et donc des enzymes nécessaires à l'hydrolyse des aliments (amylase, lipase, protéases). La non-absorption des graisses au niveau intestinal se traduit par une augmentation très importante de la stéatorée chez ces patients et par un ralentissement très sensible de la prise de poids chez les jeunes malades. Pour remédier à cela on administre à ces sujets des extraits pancréatiques de porc au moment des repas. L'efficacité thérapeutique de ces extraits pourrait être améliorée par la co-prescription de LGL comme le laissent supposer les études de synergie réalisées in vitro et in vivo (Abrams, C.K. et al., J. Clin. Invest. 73 (1984) 374-382; Levy, E. et al., Nutrition Research 11 (1991) 607-619).

Dans le brevet FR 2 603 804, et dans la demande de brevet européen correspondant EP 0 261 016, sont décrites la purification et la détermination de la séquence NH₂-terminale de la LGL. Différents procédés permettant d'extraire cette enzyme à partir d'estomacs de lapin sont également décrits dans ce brevet. Ces procédés consistent essentiellement à faire subir à des estomacs de lapins une extraction par un milieu aqueux acide, on relargue l'extrait lipasique par addition de sels hydrosolubles, puis par une filtration sur tamis moléculaire, suivie d'une séparation par chromatographie par échange d'ions, et l'on recueille une fraction d'élution contenant la lipase. La LGL purifiée obtenue par ces procédés a un poids moléculaire selon la technique de Laemmli de 49 000 daltons, dont 9 000 correspondent à des sucres et 40 000 à une protéine. Ces procédés d'obtention de la LGL purifiée, ont été développés au niveau du laboratoire et à l'échelon pilote, mais sont difficilement transposables au niveau industriel (coût, approvisionnement en matière première, changement d'échelle).

La présente invention a précisément pour but de permettre la production d'une LGL à l'échelle industrielle et à un prix de revient bien plus avantageux que ne le permettent les méthodes actuelles.

L'invention découle de la découverte faite par les inventeurs de la séquence nucléotidique de l'ARNm codant pour la LGL, après clonage de l'ADN complémentaire (ADNc) de cet ARNm à l'aide de sondes oligonucléotidiques synthétiques déduites de la séquence NH₂-terminale de la LGL décrite dans le brevet FR 2 603 804 cité ci-dessus.

La présente invention sera plus particulièrement illustrée à l'aide des figures 1 à 17 dont les légendes sont les suivantes:
- Figure 1: : Comparaison des séquences NH₂-terminales des lipase gastrique humaine, lipase linguale de rat, lipase gastrique de lapin et lipase pancréatique de porc.
- Figure 2: : Séquence des oligonucléotides synthétiques correspondant à la région NH₂-terminale de la LGL.
- Figure 3: : Mise en évidence, par hybridation avec des sondes synthétiques spécifiques de l'ARN messager codant pour la LGL après transfert de type "Northern" sur membrane de nylon des ARN polyA+ extraites de la muqueuse fundique d'estomac de lapin et séparés selon leur taille par électrophorèse sur gel d'agarose dans des conditions dénaturantes :
A. Hybridation à 37°C avec lip9, lavage à 37°C pendant 15 minutes.
B. Hybridation à 37°C avec lip9, lavage à 37°C pendant 30 minutes.
C. Hybridation à 37°C avec lip8, lavage à 42°C pendant 30 minutes.
D. Hybridation à 37°C avec lip8, lavage à 48°C pendant 30 minutes.
- Figure 4: : Schéma du clonage de l'ADNc de la LGL.
- Figure 5: : Carte de restriction du plasmide pJ0101.
- Figure 6: : Séquence nucléotidique de l'ADNc codant pour le précurseur de la LGL.
- Figure 7: : Séquence polypeptidique du précurseur de la LGL.
- Figure 8: : Carte de restriction du plasmide pRU276.
- Figure 9: : Séquence nucléotidique de la région "promoteur Tac" du plasmide pRU276.
- Figure 10: : Comparaison des séquences polypeptidiques des précurseurs de la LGL et de la LGH déduite de la séquence nucléotidique de leurs ADNc.
- Figure 11: : Structures secondaires possibles de l'extrémité 5′OH des ARNm de la LGL issus des plasmides pRGLN1.3 et pRGLN2.1.
- Figure 12: : Introduction d'un codon d'initiation ATG et de nouveaux sites de restriction dans l'ADNc de la LGL par amplification génique de type "PCR" à l'aide d'amorces synthétiques spécifiques.
- Figure 13: : Construction du plasmide pRGLN1.3.
- Figure 14: : Mutagenèse in vitro de l'ADNc de la LGL par la technique "PCR" à l'aide d'amorces oligodésoxynucléotidiques spécifiques en vue de la construction du plasmide pRGLN2.1.
- Figure 15: : Construction du plasmide pRGLN2.1.
- Figure 16: : Analyse par électrophorèse sur gel de polyacrylamide-SDS des protéines synthétisées, en absence ou en présence d'IPTG, dans E. coli W3110 iq transformée par les plasmides pRGLN1.3. et pRGLN2.1.
- Figure 17: : Immunodétection à l'aide d'anticorps spécifiques de la LGL synthétisée dans E. coli W3110 iq transformée par les plasmides pRGLN1.3. et pRGLN2.1. après transfert de type "Western" sur membrane de nylon des extraits bactériens.

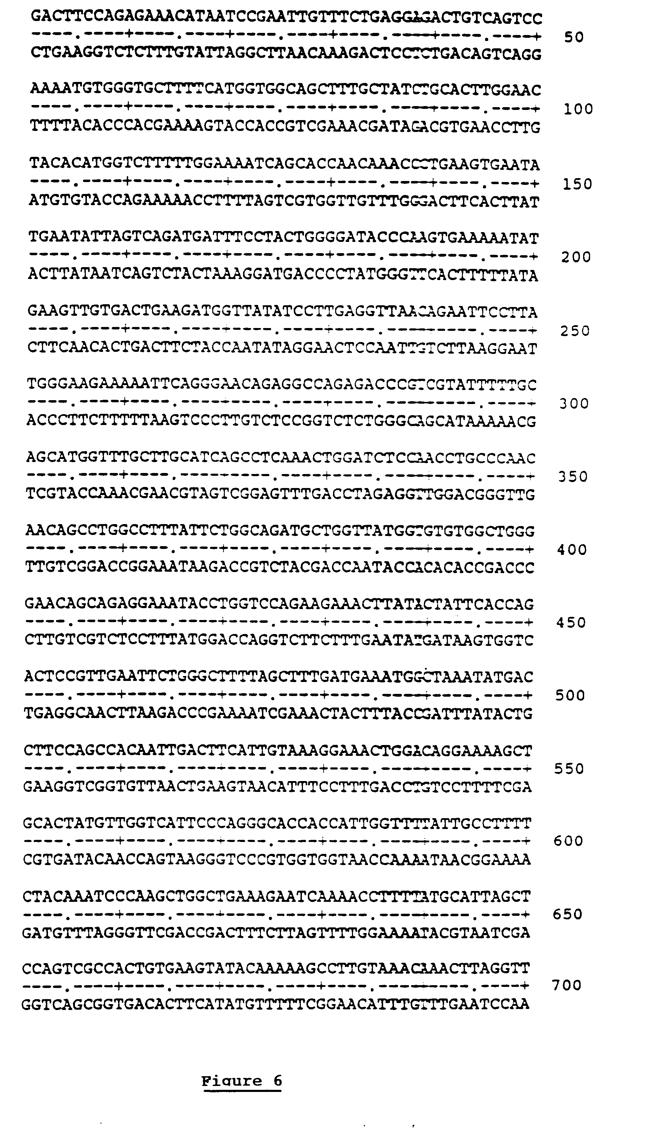

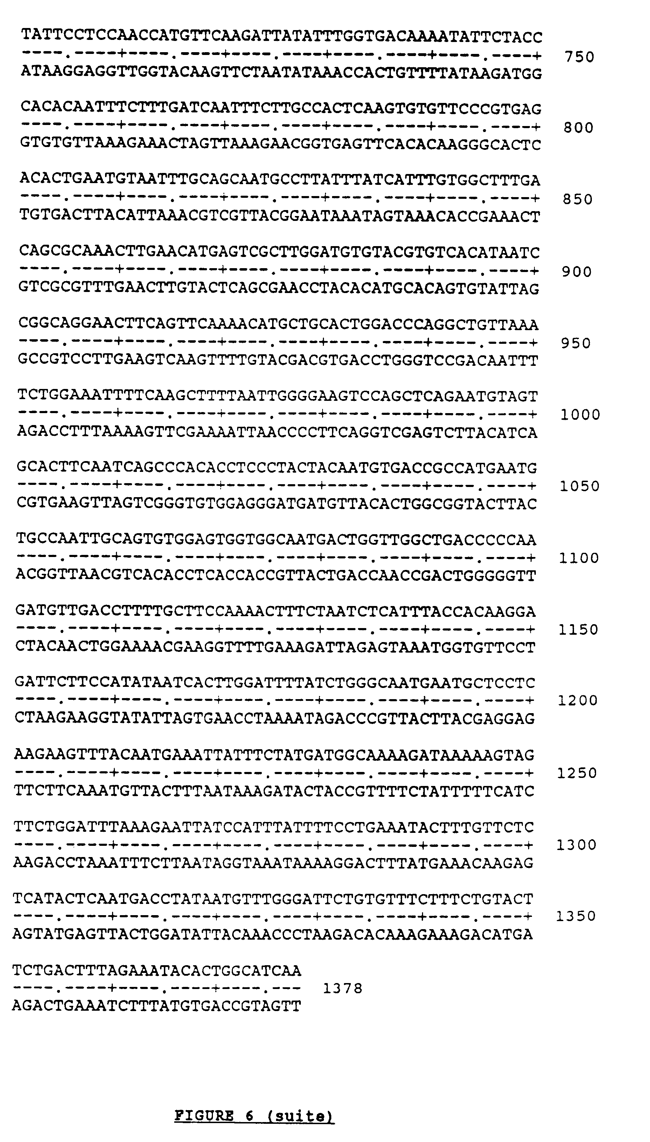

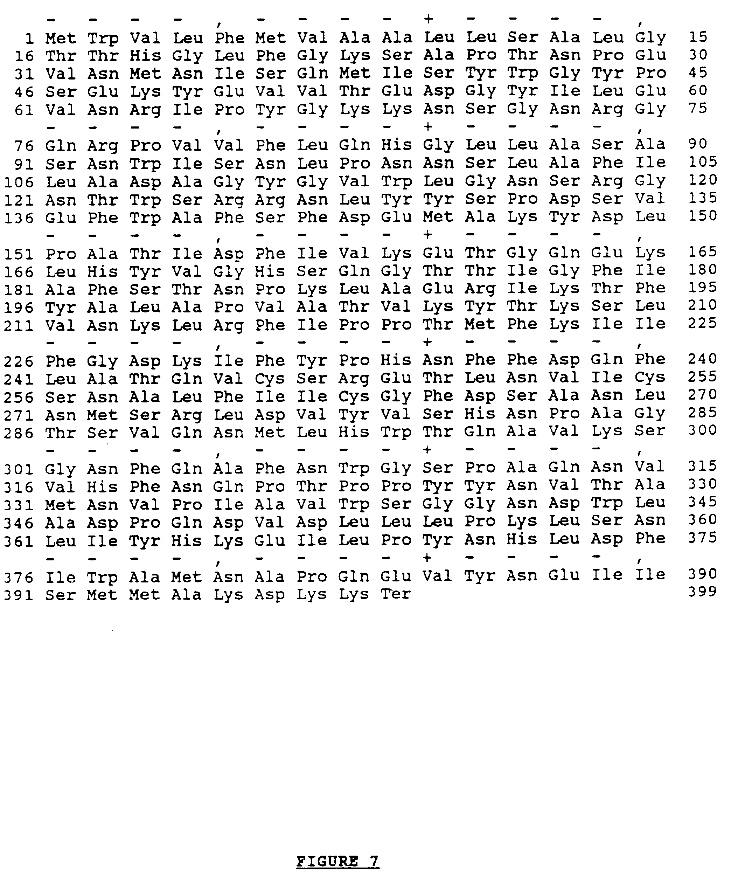

Ainsi la présente invention concerne tout acide nucléique caractérisé en ce qu'il comprend le fragment d'ADN délimité par les nucléotides situés aux positions 120 et 1247 de l'ADN représenté sur la figure 6, ce fragment d'ADN codant pour le polypeptide délimité par les acides aminés situés aux positions 23 et 398 de la séquence en acides aminés représentée sur la figure 7, ce polypeptide correspondant à la LGL mature.

L'invention concerne également tout acide nucléique tel que décrit ci-dessus, comprenant en amont du fragment d'ADN susmentionné, le fragment d'ADN délimité par les nucléotides situés aux positons 111 et 119 de l'ADN représenté sur la figure 6, et codant pour la séquence en acides aminés Leu-Phe-Gly.

Les acides nucléiques susmentionnés peuvent également comprendre en amont du fragment d'ADN délimité par les nucléotides situés aux positions 120 et 1247, et de celui délimité par les nucléotides situés aux positions 111 et 1247 de la figure 6, un fragment d'ADN codant pour une méthionine.

L'invention vise également les fragments d'ADN susmentionnés présentant en aval de la position 1247 un codon STOP, notamment celui constitué de la séquence délimitée par les nucléotides situés aux positions 1248 et 1250 de la figure 6.

L'invention a également pour objet tout acide nucléique caractérisé en ce qu'il comprend le fragment d'ADN délimité par les nucléotides situés aux positions 54 et 1247 de l'ADN représenté sur la figure 6, ce fragment d'ADN codant pour le polypeptide de 398 acides aminés représenté sur la figure 7, ce polypeptide correspondant au précurseur de la lipase gastrique de lapin. En effet, comme il l'a déjà été précisé ci-dessus, la LGL est synthétisée sous forme d'un précurseur constitué d'un peptide signal de 22 AA précédant la séquence polypeptidique de la protéine mature.

D'une manière générale, l'invention concerne tout acide nucléique caractérisé en ce qu'il comprend en amont d'un des fragments d'ADN susmentionnés, une séquence signal codant pour un peptide signal.

Par opposition aux acides nucléiques double brin cités ci-dessus, l'invention vise également les acides nucléiques simple brin constitués de l'une ou l'autre des deux séquences nucléotidiques complémentaires constituant les fragments d'ADN susmentionnés.

L'invention concerne également tout acide nucléique susceptible de s'hybrider avec un acide nucléique simple brin tel que décrit ci-dessus, notamment dans les conditions d'hybridation citées dans la description détaillée qui suit du clonage de l'ADNc de la LGL selon l'invention.

Tout acide nucléique codant pour un polypeptide selon l'invention et dont la séquence nucléotidique diffère en fonction de la dégénérescence du code génétique des séquences nucléotidiques susmentionnées, entre également dans le cadre de la présente invention.

L'invention a également pour objet tout acide nucléique recombinant caractérisé en ce qu'il comprend un acide nucléique tel que décrit ci-dessus selon l'invention, inséré dans une séquence nucléotidique hétérologue vis à vis du susdit acide nucléique.

A ce titre l'invention a plus particulièrement pour objet tout acide nucléique recombinant comprenant un promoteur situé en amont de l'acide nucléique selon l'invention, et sous le contrôle duquel la transcription dudit acide nucléique est susceptible d'être effectuée, ainsi qu'une séquence codant pour des signaux de terminaison de la transcription située en aval dudit acide nucléique.

L'invention concerne également tout vecteur recombinant, notamment du type plasmide, cosmide ou phage, caractérisé en ce qu'il contient un acide nucléique recombinant tel que décrit ci-dessus, inséré en l'un de ses sites non essentiels pour sa réplication.

Les vecteurs recombinants selon l'invention sont avantageusement caractérisés en ce qu'ils contiennent en l'un de leurs sites non essentiels pour leur réplication, des éléments nécessaires pour promouvoir et contrôler l'expression d'un acide nucléique selon l'invention dans un hôte cellulaire, et plus particulièrement un promoteur reconnu par les polymérases de l'hôte cellulaire, notamment un promoteur inductible.

L'invention vise également tout hôte cellulaire, de type procaryote ou eucaryote, transformé par un vecteur recombinant tel que décrit ci-dessus, et comprenant les éléments de régulation permettant l'expression d'un acide nucléique selon l'invention.

A titre d'exemples de cellules hôtes susceptibles d'être transformées par un vecteur recombinant selon l'invention, on peut citer les cellules de mammifères telles que les cellules COS ou CHO, les levures telles que saccharomyces cerevisiae ou kluyveromyces lactis, ainsi que les bactéries telles que E. coli (bactérie Gram négatif) ou B. subtilis (bactérie Gram positif), ou encore les champignons filamenteux comme Aspergillus niger.

L'invention a également pour objet des amorces d'ADN (ou d'ARN) utilisables pour la synthèse des acides nucléiques selon l'invention par la technique d'amplification en chaîne de l'ADN, ci-après désignée par technique PCR (Polymerase Chain réaction). Cette technique est plus particulièrement décrite dans les brevets américains N° 4, 683, 202 et n° 4, 683, 195, ainsi que dans le brevet européen n° 200.362. Les amorces selon l'invention sont avantageusement constituées d'environ 10 à 40 nucléotides correspondant aux extrémités 3′ et 5′ de l'un ou l'autre des deux brins constituant les fragments d'ADN susmentionnés.

L'invention vise également des sondes nucléotidiques issues de l'un ou l'autre des deux brins constituant les fragments d'ADN susmentionnés de l'invention, ainsi que l'utilisation de ces sondes, notamment pour la détection dans un échantillon biologique de la présence éventuelle de LGL. Avantageusement, les sondes de l'invention sont constituées d'environ 14 à environ 40 nucléotides. La détection de la présence de LGL dans un échantillon, est de préférence réalisée après amplification du nombre de copies des gènes ou des ARNm codant pour la LGL susceptibles d'être présents dans cet échantillon, à l'aide des amorces indiquées ci-dessus.

A ce titre, l'invention concerne également un kit pour la mise en oeuvre de la méthode de détection susmentionnée, comprenant:
- le cas échéant, des amorces telles que décrites ci-dessus, ainsi que les réactifs pour la constitution d'un milieu propice à la réalisation de l'amplification de la séquence d'ADN ou d'ARN codant pour la LGL,
- une sonde nucléotidique telle que décrite ci-dessus, le cas échéant marquée, notamment de manière radioactive ou enzymatique, ainsi que les réactifs pour la constitution d'un milieu propice à la réalisation de l'hybridation entre la sonde et la séquence d'ADN ou d'ARN susmentionnée,
- les réactifs permettant la détection de la sonde hybridée avec ladite séquence.

Avantageusement les sondes nucléotidiques de l'invention sont susceptibles de s'hybrider à la fois avec la séquence d'ADN ou d'ARN codant pour la LGL et avec celle codant pour la lipase gastrique humaine (LGH). De telles sondes sont utilisables pour la mise en oeuvre d'une méthode de détection in vitro de la présence éventuelle de LGH dans un échantillon biologique susceptible de contenir cette dernière. Une telle méthode de détection est réalisée de la manière indiquée ci-dessus, et permet le diagnostic in vitro de pathologies liées à la surproduction, ou à l'inverse, à l'insuffisance, voire l'absence, de production de lipase gastrique dans l'organisme, et plus particulièrement de pathologies telles que la mucoviscidose, et l'insuffisance pancréatique exocrine.

L'invention a également pour objet les polypeptides recombinants correspondant selon le code génétique universel aux acides nucléiques selon l'invention décrits ci-dessus, ou tout fragment de ces polypeptides recombinants, ou tout polypeptide modifié par substitution et/ou addition et/ou suppression d'un ou plusieurs acides aminés de ces polypeptides recombinants, ces fragments ou polypeptides modifiés conservant les propriétés enzymatiques des polypeptides recombinants susmentionnés.

Il faut entendre par "polypeptides recombinants" toute molécule possédant une chaîne polypeptidique susceptible d'être produite par voie du génie génétique, par transcription et traduction d'une séquence d'ADN correspondante sous le controle d'éléments de régulation appropriés à l'intérieur d'un hôte cellulaire efficace. Par conséquent, l'expression "polypeptides recombinants" n'exclut pas la possibilité que ces polypeptides comprennent d'autres groupes, tels que des groupes glycosylés.

Le terme "recombinant" implique le fait que le polypeptide a été produit par voie du génie génétique, plus particulièrement en raison du fait que ce polypeptide résulte de l'expression dans un hôte cellulaire de séquences d'acides nucléiques qui ont été préalablement introduites dans le vecteur d'expression utilisé dans ledit hôte.

Toutefois, il doit être entendu que cette expression n'exclut pas la possibilité que le polypeptide soit produit par un procédé différent, par exemple par synthèse chimique classique selon les méthodes classiquement utilisées pour la synthèse des protéines, ou par clivage de molécules de plus grande taille de chaîne en acides aminés.

L'invention vise également les polypeptides susmentionnés sous forme biologiquement pure. Il faut entendre par l'expression "biologiquement pure" d'une part un degré de pureté permettant au polypeptide recombinant d'être utilisé pour la production de compositions pharmaceutiques, et, d'autre part, l'absence de contaminants, plus particulièrement de contaminants naturels.

A ce titre, l'invention concerne plus particulièrement:
- le polypeptide délimité par les acides aminés situés aux positions 23 et 398 de la séquence en acides aminés représentée sur la figure 7, et correspondant à la LGL mature telle qu'obtenue par voie du génie génétique (encore désignée par l'expression LGL recombinante), et dont le poids moléculaire varie d'environ 44.000 à environ 50.000 daltons, suivant que l'hôte, dans lequel elle est produite, effectue des modifications post-traductionnelles sur la chaîne polypeptidique de cette LGL,
- le polypeptide constitué de la séquence en acides aminés représentée susmentionnée et d'une séquence Leu-Phe-Gly située en amont de cette dernière,
- les polypeptides sus-mentionnés dont les séquences en acides aminés sont précédées d'une méthionine,
- le polypeptide de 398 acides aminés représenté sur la figure 7, et correspondant au précurseur de la LGL tel qu'obtenu par voie du génie génétique; l'analyse du clone contenant l'ADNc codant pour le précurseur de la LGL a permis de déterminer que ce dernier est constitué de 398 acides aminés et qu'il possède un poids moléculaire de 44913 daltons.

Les polypeptides selon l'invention sont davantage caractérisés:
- en ce qu'ils possèdent sous leur forme purifiée une activité lipasique (ou encore activité lipolytique correspondant à l'hydrolyse de triglycérides à chaînes longues) spécifique égale ou supérieure à 1000 U/mg de protéine déterminée selon la méthode de Gargouri,
- leur activité lipolytique maximale est obtenue à des valeurs de pH allant de 3,5 à 6,
- leur activité lypolytique spécifique est inchangée après incubation durant une heure à pH 2 et à 37°C,
- leur activité lypolytique spécifique déterminée n'est que de 20 % après incubation durant 12 heures à 37° C dans des milieux de pH 3 à pH 6.

La présente invention concerne également un procédé de préparation d'un polypeptide tel que décrit ci-dessus, ce procédé comprenant la succession d'étapes suivantes:
- la mise en culture d'un hôte cellulaire, transformé par un vecteur recombinant tel que décrit ci-dessus, dans un milieu de culture approprié, et
- la récupération du polypeptide produit par ledit hôte cellulaire, soit directement à partir du susdit milieu de culture, lorsque la séquence codant pour ledit polypeptide est précédée d'une séquence signal et que l'hôte cellulaire est capable de sécréter le polypeptide dans le milieu de culture (notamment dans le cas des cellules eucaryotes et des levures), soit après lyse de l'hôte cellulaire (notamment dans le cas des bactéries).

Le cas échéant, l'étape de récupération est suivie d'une étape de purification du polypeptide récupéré, et, notamment après la récupération par lyse de la bactérie, le cas échéant par une étape de solubilisation du polypeptide, puis de sa renaturation.

Les agents et les techniques de solubilisation des polypeptides obtenus sous forme d'inclusion sont bien connus de l'homme de l'art. Essentiellement les agents solubilisants sont l'urée, des halogénures d'ammonium quaternaires comme le chlorure de guanidinium ou le chlorure de cétyltriméthylammonium qui sont utilisés dans des procédures expérimentales telles que celles décrites par N.K. Puri et coll. dans Biochem. J (1992) 285, 871-879.

Avantageusement, comme il l'a été déjà précisé plus haut, les séquences nucléotidiques codant pour les polypeptides dont la production est recherchée, et insérées dans l'acide nucléique du vecteur utilisé pour transformer les cellules hôtes, sont précédées d'une séquence signal permettant ainsi la sécrétion des polypeptides produits hors des cellules hôtes et leur récupération directement à partir du milieu de culture sans avoir à procéder à la lyse desdites cellules hôtes.

A titre d'exemple, la synthèse de la LGL mature dans des cellules de mammifère telles que les cellules COS ou les cellules CHO, pourra être obtenue en insérant l'acide nucléique codant pour le précurseur LGL dans un vecteur d'expression approprié. La présence du segment d'ADN codant pur le peptide signal, à savoir le segment d'ADN délimité par les nucléotides situés aux positions 54 et 119 de la figure 6, permettra à la machinerie cellulaire de glycosyler et sécréter la LGL dans le milieu de culture sous forme biologiquement active.

Pour faire produire et sécréter la LGL par une levure telle que Saccharomyces cerevisiae ou Kluyveromyces lactis, il sera préférable de remplacer, dans le cDNA, le segment d'ADN codant pour le peptide signal de la LGL par un fragment d'ADN codant pour un peptide signal de protéine de levure. L'ADNc recombinant ainsi obtenu sera alors introduit dans un vecteur d'expression spécifique de l'hôte considéré. De tels systèmes d'expression sont maintenant relativement courants. Par exemple, on peut citer l'expression de la sérum albumine humaine (Brevet Européen n° 0361 991 A2) ou de la chymosine de veau (Van den Berg J.A. et al., Biotechnology 8 (1990) 135-139).

Escherichia coli est une bactérie Gram négatif pourvue d'une paroi chez qui les phénomènes de sécrétion de protéines dans le milieu de culture sont extrêmement réduits. Un certain nombre de protéines s'accumulent dans le périplasme bactérien grâce à la présence de signaux similaires aux séquences signal des protéines eucaryotes. Parmi celles-ci, on peut citer les produits des gènes phoA et malE par exemple. Certaines régions de ces gènes ont été utilisées pour produire des protéines hétérologues dans l'espace périplasmique de E. coli. Cependant, la synthèse dans le cytoplasme de protéines étrangères demeure le système le mieux connu et le plus utilisé chez E. coli.

Le respect de certaines règles déduites de l'expérience lors de la réalisation des constructions de plasmides permet d'optimiser le niveau d'expression des protéines d'intérêt.

Dans un premier temps, il conviendra de placer l'ADNc codant pour la partie mature de la LGL, c'est-à-dire dépourvu du segment codant pour le peptide signal, derrière un promoteur puissant bactérien ou phagique. Pour éviter des problèmes de toxicité éventuelle de la protéine étrangère dans la bactérie, on choisira de préférence un promoteur inductible par un agent chimique (promoteurs Lac ou Trp) ou par un agent physique tel qu'un changement de température (promoteur PL et répresseur cI 857). L'ADNc devra être contigu, dans sa région 5′ terminale à une séquence ATG spécifiant l'initiation de la synthèse protéique sur l'ARN messager. Cet ATG initiateur devra lui-même être précédé d'une séquence de 4 à 6 nucléotides riche en purines, située à une distance de 6 à 12 paires de bases et correspondant, sur l'ARN messager, au site de fixation des ribosomes ou séquence de Shine et Dalgarno.

La séquence du segment d'ADN situé entre la région de Shine et Dalgarno et l'ATG initiateur pourra être modifiée dans sa composition de manière à réduire les éléments de structure secondaire autour du codon d'initiation AUG sur l'ARN messager. Une fois les modifications nécessaires apportées, le vecteur sera introduit dans un hôte approprié.

L'invention concerne les anticorps eux-mêmes dirigés contre les polypeptides de l'invention, et plus particulièrement ceux dirigés contre la LGL et susceptibles de reconnaître également la LGH. De tels anticorps peuvent être obtenus par immunisation d'un animal avec ces polypeptides suivie de la récupération des anticorps formés.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des polypeptides purifiés de l'invention, d'une part et des cellules d'un myélome approprié d'autre part, et d'être sélectionné, par sa capacité à produire des anticorps monoclonaux reconnaissant le polypeptide initialement mis en oeuvre pour l'immunisation des animaux, ainsi que la LGH.

L'invention vise également l'utilisation de ces anticorps pour la mise en oeuvre d'une méthode de détection ou de dosage de la LGL ou de la LGH dans un échantillon biologique susceptible de les contenir.

L'invention concerne plus particulièrement l'utilisation de ces anticorps pour la mise en oeuvre d'une méthode de diagnostic in vitro de pathologies liées à la surproduction, ou à l'inverse, à l'insuffisance, voire l'absence de production de lipase dans l'organisme, et plus particulièrement de pathologies telles que la mucoviscidose, et l'insuffisance pancréatique exocrine.

Cette méthode de diagnostic in vitro, réalisée à partir d'un échantillon biologique prélevé chez un patient, comprend une étape de mise en présence de cet échantillon avec des anticorps selon l'invention dans des conditions permettant la réaction immunologique entre ces anticorps et la LGH susceptible d'être présente dans cet échantillon, suivie d'une étape de détection des éventuels complexes anticorps-LGH formés lors de l'étape précédente.

A ce titre l'invention concerne également un kit pour la mise en oeuvre d'une méthode de détection ou de diagnostic in vitro susmentionnée, comprenant:
- des anticorps tels que décrits ci-dessus, avantageusement marqués de manière radioactive ou enzymatique, ainsi que les réactifs pour la constitution d'un milieu propice à la réalisation de la réaction immunologique entre ces anticorps et la LGH,
- les réactifs permettant la détection des complexes immunologiques formés entre ces anticorps et la LGH.

L'invention concerne également l'utilisation d'un ou plusieurs polypeptides décrits ci-dessus, et plus particulièrement la LGL recombinante, pour l'obtention de compositions pharmaceutiques, utilisables notamment par voie orale, destinées à faciliter l'absorption des graisses animales ou végétales (principalement au niveau intestinal) ingérées par un individu sain ou atteint d'une ou plusieurs pathologies affectant ou non le taux de production de lipase gastrique. Notamment, de telles compositions sont avantageusement utilisées chez les individus subissant un traitement médical altérant le mécanisme d'absorption des graisses, ou encore chez les personnes âgées.

L'invention vise plus particulièrement l'utilisation d'un, ou plusieurs polypeptides décrits ci-dessus, et plus particulièrement la LGL recombinante, pour l'obtention de médicaments destinés au traitement des pathologies liées à l'insuffisance, voire l'absence, , de production de lipase dans l'organisme, et plus particulièrement de pathologies telles que la mucoviscidose, et l'insuffisance pancréatique exocrine.

L'invention a également pour objet des compositions pharmaceutiques comprenant au moins un polypeptide selon l'invention, -le cas échéant en combinaison avec tout autre polypeptide à activité lipasique-, en association avec un véhicule pharmaceutiquement acceptable.

L'invention concerne également l'utilisation des polypeptides tels que décrits ci-dessus selon l'invention ou de toute autre lipase gastrique de mammifères et dérivés, pour la mise en oeuvre de réactions de bioconversions enzymatiques (telles que hydrolyses, trans-estérifications enzymatiques), notamment sous forme immobilisée sur un support solide.

L'invention sera plus particulièrement illustrée à l'aide de la description détaillée qui suit de la construction de vecteurs recombinants selon l'invention et de leur utilisation pour la production de LGL.

Une préparation d'ARN a été réalisée à partir de muqueuse isolée de la région fundique d'estomac de lapin. Les ARN messagers isolés par chromatographie d'affinité, sur colonne d'oligo-dT cellulose ont été convertis en ADN complémentaire (ADNc) grâce à l'utilisation d'enzymes particulières: la reverse transcriptase du Virus du Sarcome de Rous et la DNA polymérase I de E. coli (fragment de Klenow). Cet ADNc a été introduit dans le vecteur pUC18 après certaines modifications et les molécules recombinantes ont été utilisées pour transformer la bactérie E. coli, MM294. Les clones transformants ont été criblés par hybridation in situ à l'aide de sondes synthétiques oligonucléotidiques radioactives déduites de la séquence NH₂-terminale de la LGL.

Les colonies bactériennes correspondant à un signal positif lors des expériences d'hybridation, ont été isolées et l'ADN plasmidique présent dans leur cytoplasme amplifié et purifié.

Après purification, le clone pJ0101, contenant un ADNc de 1378 paires de bases (pb) a été sélectionné et séquencé. L'analyse de la séquence nucléotidique de cet ADNc a permis de mettre en évidence une phase ouverte de lecture de 1194 nucléotides (NT) correspondant à une protéine de 398 AA et d'un poids moléculaire de 44913 daltons.

La comparaison avec les séquences nucléotidiques des ADNc des autres lipases préduodénales (Docherty, A.P.J. et al. (1985) op.cit.; Bodmer, M.W. et al. (1987) op.cit.) fait apparaître une homologie de 87,9% avec la LGH et de 79% avec la LLR (lipase linguale de rat) dans les régions codantes.

### Techniques générales de clonage:

Les méthodes classiques de biologie moléculaire telles que la purification des ARN messagers, l'extraction et la purification d'ADN plasmidique, la digestion par des enzymes de restriction, l'électrophorèse sur gel d'agarose ou de polyacrylamide, l'électroélution de gel d'agarose de fragments d'ADN, la transformation dans E. coli, sont décrites dans la littérature (Maniatis, T; et al., "Molecular cloning : a laboratory manual, Second Edition", Cold Spring Harbor Laboratory, Cold Spring Harbor, N.Y., 1989; Ausubel, F.M. et al. (eds.), "Current Protocols in Molecular Biology", John Willey and Sons, New-York, 1987).

Les enzymes sont obtenues auprès des sociétés Boehringer ou New England Biolabs et utilisées dans les conditions préconisées par les fournisseurs.

Les fragments d'ADN destinés à être assemblés sont séparés selon leur taille par électrophorèse sur gel d'agarose 1 %, purifiés par électroélution et précipités par l'éthanol. La ligature des fragments d'ADN s'effectue en présence de T4DNA ligase à 4°C ou à 16°C dans un tampon approprié selon que les morceaux à assembler possèdent des extrémités franches ou cohésives.

Le séquençage de l'ADN s'effectue selon la méthode des didésoxynucléotides (Sanger, F. et al., Proc. Nat. Acad. Sci. USA. 74 (1977) (5463-5467) en utilisant un kit "T7 Sequencing" (Pharmacia).

L'amplification enzymatique de fragments d'ADN spécifiques s'effectue selon la méthode de "Polymerasecatalysed Chain réaction" ou PCR (Saiki, R.K. et al., Science 230 (1985) 1350-1354) à l'aide d'un appareil PREM III LEP Scientific.

Les oligonucléotides utilisés comme sondes radiomarquées ou comme amorces dans les réactions de PCR ou de séquençage sont synthétisées à l'aide d'un synthétiseur d'ADN modèle 391 PCR-MATE (Applied Biosystems) et purifiés par chromatographie haute pression en phase liquide avant leur utilisation.

Les molécules d'ADN recombinantes sont utilisées pour transformer des cellules compétentes des souches suivantes de E. coli:
- MM294 [F-, endA1, hsdR17 (rₖ-mₖ+), supE44, thi-1, relA1?],
- MC1061[F-,araDA139, (ara-leu)7579, (Lac)X74, ga1U, galK, hsdR2 (rₖ-mₖ+), mcrB1, rpsL(Str^{r})] ou
- W3110 i^{q} [F' TraD36, LacI^{q}, (lac Z) M15,pro⁺].

L'ADN plasmidique est extrait des transformants bactériens résistants à l'ampicilline selon un protocole dérivé de la méthode de lyse alcaline décrite par Birnboim et Doly (Birnboim, H.C. and Doly, J., Nucl. Ac. Res. 7 (1979) 1513-1523).

L'immunodétection de la lipase gastrique de lapin synthétisée dans la bactérie E. coli W3110 i^{q}, après addition d'IsopropylThioGalactopyranoside (IPTG) au milieu de culture, s'effectue par une méthode d'immunoempreinte sur membrane de nylon en utilisant un anticorps de cobaye anti-LGL et le kit de révélation à la peroxydase ImmmunoPure ABC (Pierce).

### Exemple n° 1 : Clonage d'un ADNc codant pour la lipase gastrique de lapin

1.1. Isolement et purification des ARN messagers de muqueuse fundique d'estomac de lapin.

Après broyage des tissus dans un tampon contenant du chlorure de lithium et de l'urée (Auffray, C. and Rougeon F., Eur. J. Biochem. 107 (1980) 303-314), l'ARN total est séparé de l'ADN par précipitation sélective au chlorure de lithium. Les protéines contaminant l'ARN sont alors éliminées par extraction phénolique. Les ARN messagers, polyadénylés à leur extrémité 3'OH, sont séparés des ARN ribosomaux par chromatographie sur colonne d'oligodT-cellulose (Maniatis, T. et al., déjà cité). On obtient ainsi environ 8 mg d'ARN total par gramme de tissu, contenant 1 à 3 % d'ARN messager polyadénylé.
1.2. Mise en évidence de l'ARN messager codant pour la LGL dans la préparation d'ARN messager extraite de muqueuse fundique d'estomac de lapin.

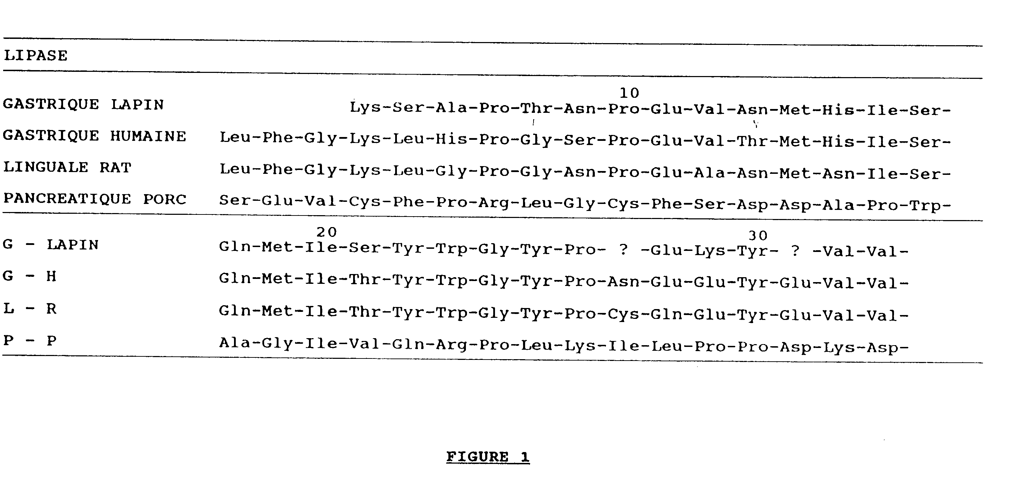

La lipase gastrique de lapin a été purifiée à l'homogénéité et sa séquence polypeptidique NH₂-terminale déterminée (Moreau, H. et al., Biochem. Biophys. Act. 960 (1988) 286-293). La figure 1 présente la comparaison des séquences polypeptidiques aminoterminales de la lipase gastrique de lapin, de la lipase gastrique humaine, de la lipase linguale de rat et de la lipase pancréatique de porc. Les séquences des trois lipases préduodénales n'ont aucune homologie avec la lipase pancréatique de porc. A l'exception du résidu 21 (Ser au lieu de Thr), les séquences des trois lipases préduodénales sont identiques, du résidu Met 14 au résidu Pro 26. Des sondes "anti-sens" peuvent être déduites de la séquence amino-terminale de la LGL en tenant compte de la dégénérescence du code génétique.

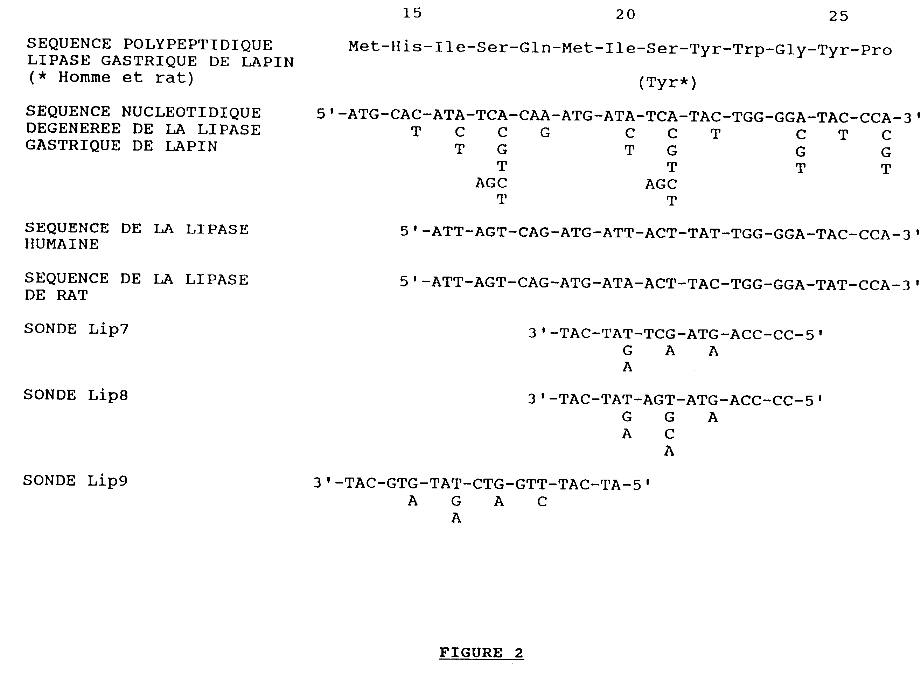

La séquence des sondes Lip7, Lip8 et Lip9, est présentée dans la figure 2.

Afin de vérifier la spécificité des sondes, des expériences d'hybridation de type "Northern" sont réalisées. Six échantillons de 10 µg d'ARN messager d'estomac de lapin sont dénaturés à 60°C, en présence de glyoxal et de DMSO, avant d'être séparés selon la taille, par électrophorèse, dans un gel d'agarose 1 % en tampon phosphate 10 mM pH=7 (Thomas, P., Proc. Nat. Acad. Sci. USA. 77 (1980) 5201-5205).

Après électrophorèse, l'ARN messager est transféré sur des membranes de nylon (Biodyne PALL) selon le protocole préconisé par le fournisseur.

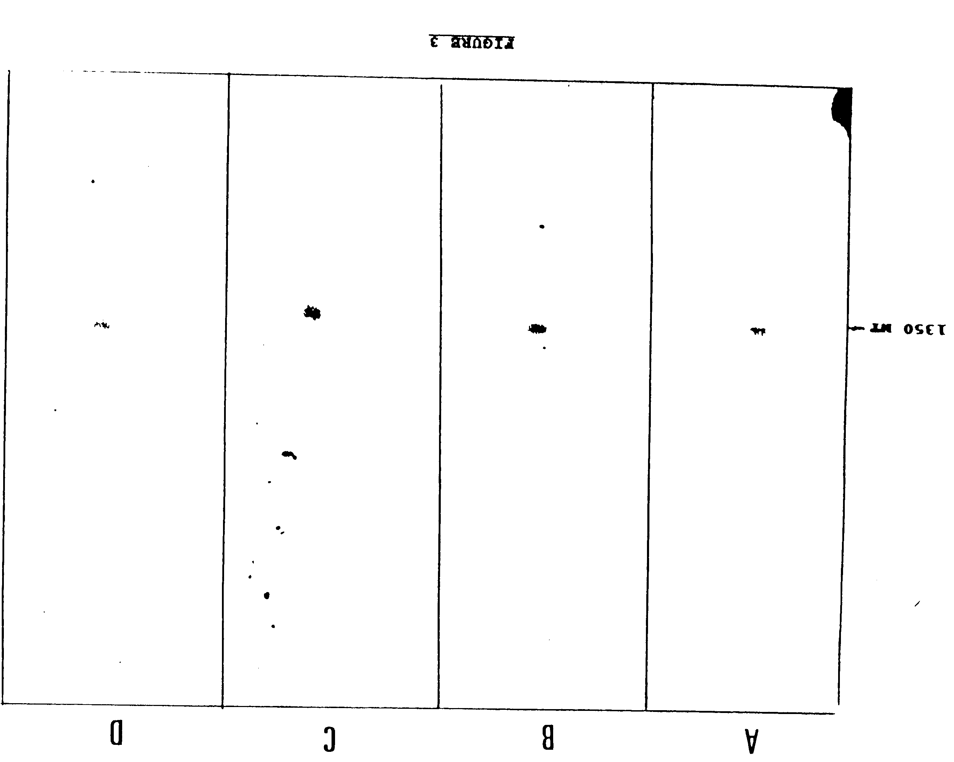

Les oligonucléotides Lip7, Lip8 et Lip9 sont marqués à leur extrémité 5′OH à l'aide de 32p-γ-ATP et de T4 polynucléotide kinase. Les membranes précédemment obtenues sont hybridées individuellement pendant 36 heures à 37°C dans un tampon 6 X SSC - 1 X Denhardt's - 0,05 % pyrophosphate de sodium contenant 25 ng/ml des sondes radioactives Lip7, Lip8 ou Lip9 (Ausubel, F. et al. (eds), déjà cité). Les membranes ont été lavées dans un tampon 6X SSC - 0,05% de pyrophosphate de sodium à différentes températures qui tiennent compte de la taille de la sonde et de son contenu en (G+C). La sonde Lip7 n'a pas permis de détecter un signal d'hybridation par autoradiographie. Les résultats obtenus avec les sondes Lip8 et Lip9 sont présentés dans la Figure 3. Un ARN d'environ 1350 nucléotides s'hybride avec les sondes Lip8 et Lip9. La taille de cet ARN correspond à celles des ARNm codant pour la lipase linguale de rat (Docherty, A.J.P. et al., déjà cité) et pour la lipase gastrique humaine (Bodmer, M.W. et al. déjà cité).
1.3. Synthèse d'ADN complémentaire de l'ARN messager d'estomac de lapin et insertion dans le vecteur pUC18.

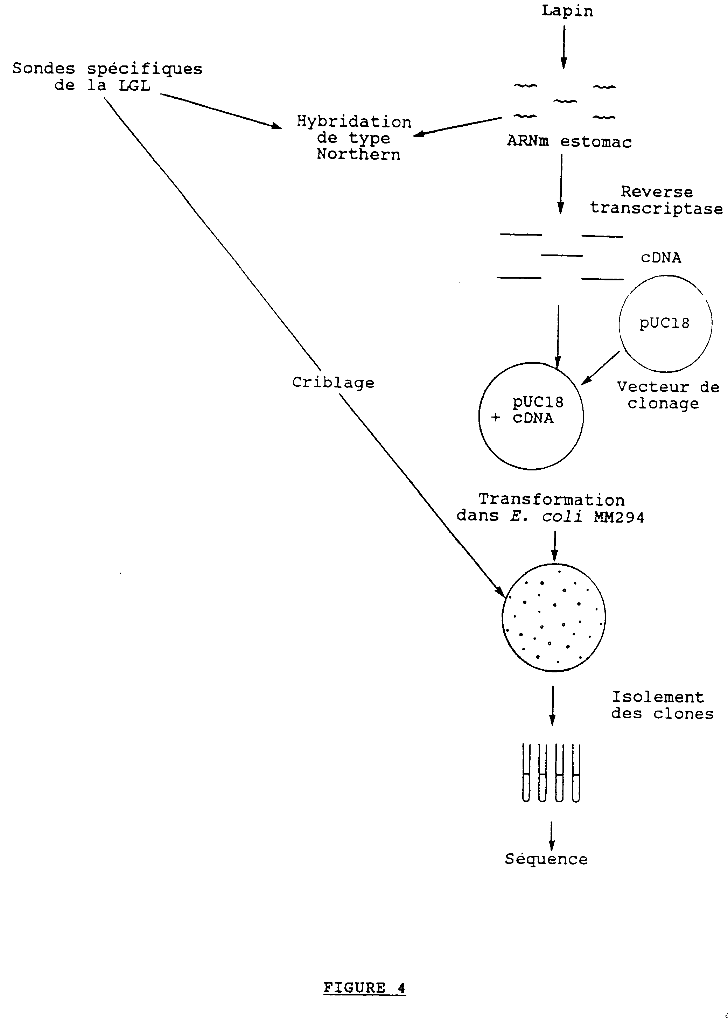

La synthèse d'ADNc double brin est réalisée à partir de 6µg d'ARN polyA+. Une fraction de cet ADN est insérée dans le vecteur pUC18 par le biais de queues oligodC et oligodG ajoutées respectivement sur l'ADNc et sur le vecteur préalablement linéarisé par l'enzyme PstI (Gubler, U. and Hoffman, B.J., Gene 25 (1983) 263-269). L'ensemble de ces étapes est présenté dans la figure 4.

Les molécules hybrides sont utilisées pour transformer des bactéries compétentes E. coli MM294. La sélection des transformants s'effectue en étalant le produit de la transformation sur un milieu nutritif solide (LB-Agar) contenant de l'ampicilline à 50 mg/litre.
1.4. Isolement de l'ADNc codant pour la LGL.

Les colonies bactériennes sont transférées sur des membranes de nylon (Biodyne PALL) et lysées selon un procédé recommandé par le fournisseur. Cette opération a pour effet de dénaturer et de fixer sur la membrane l'ADN bactérien et plasmidique contenu dans les colonies.

Après plusieurs lavages dans un tampon 3X SSC - 0,1 % SDS, à température ambiante puis à 65°C, les filtres sont préhybridés à 37°C dans un tampon 6X SSC - 1X Denhardt's - 0,05 % pyrophosphate de sodium - 0,5 % SDS.

L'hybridation s'effectue pendant 16 heures à 37°C dans un tampon 6X SSC - 1 Denhardt's - 0,05 % pyrophosphate de sodium contenant la sonde Lip8 marquée au 32P à son extrémité 5′ à 10 ng/ml.

Les filtres sont lavés deux fois dans un tampon 6X SSC - 0,05% pyrophosphate de sodium à température ambiante avant d'être placés en autoradiographie pendant 48 heures.

Un deuxième criblage est effectué sur deux séries de filtres avec les sondes Lip8 et Lip9 pour les colonies ayant donné un signal d'hybridation positif lors du premier criblage.

Quatre clones ont été sélectionnés après le deuxième criblage. La coupure par l'enzyme de restriction PstI de l'ADN plasmidique de ces clones montre qu'ils contiennent tous un plasmide ayant un insert PstI-PstI de 1,35 Kb. L'un des plasmides, pJ0101, est préparé à partir d'un litre de culture bactérienne en vue de son analyse détaillée et de son séquençage.

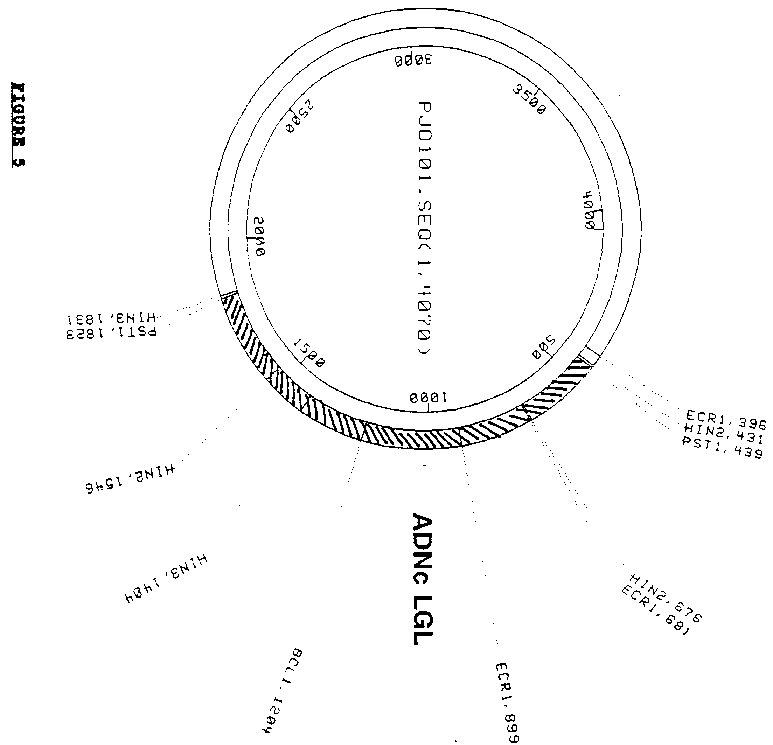

La carte de restriction du plasmide pJ0101 est présentée dans la figure 5. Le séquençage de l'insert ADNc est réalisé sur l'ADN double brin par la méthode de Sanger (Sanger, F. et al., Proc. Natl. Acad. Sci. USA. 74 (1977) 5463-5467).

La séquence complète de l'ADNc comporte 1378 nucléotides et est présentée dans la figure 6. Une phase ouverte de lecture allant du nucléotide 54 au nucléotide 1247 code pour une protéine de 398 AA. La séquence de cette protéine est rapportée dans la figure 7. Cette protéine possède 84,4 % d'homologie avec la lipase gastrique humaine (Bodmer et al. (1987) op. cit.) et 75 % avec la lipase linguale de rat (Docherty et al. (1985) op. cit.). Les résidus 23 à 52 correspondent à la séquence NH₂-terminale déterminée sur la LGL purifiée (Moreau, H. et al. (1988) déjà cité). La position 34 correspond à un résidu Asn et non His comme cela avait été précédemment décrit. Les positions 46 et 50 non identifiées préalablement, correspondent respectivement à des résidus Ser et Glu. La LGL est donc synthétisée sous forme d'un précurseur constitué d'un peptide signal amino-terminal de 22 AA précédant la chaîne polypeptidique de l'enzyme.

### Exemple n° 2: Construction des plasmides pour exprimer la LGL dans Escherichia coli.

2.1. Choix du vecteur d'expression.

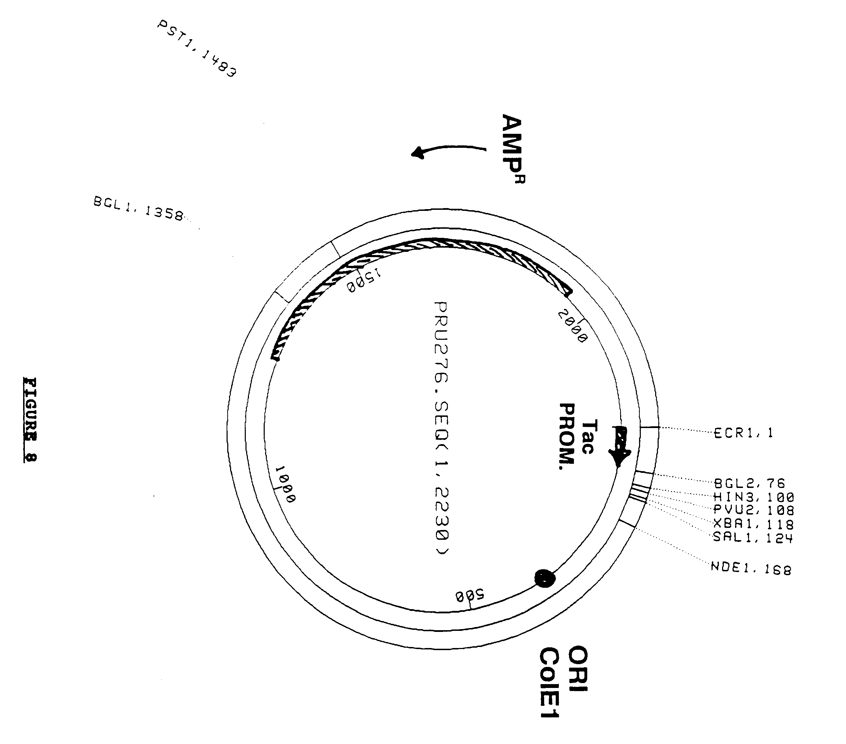

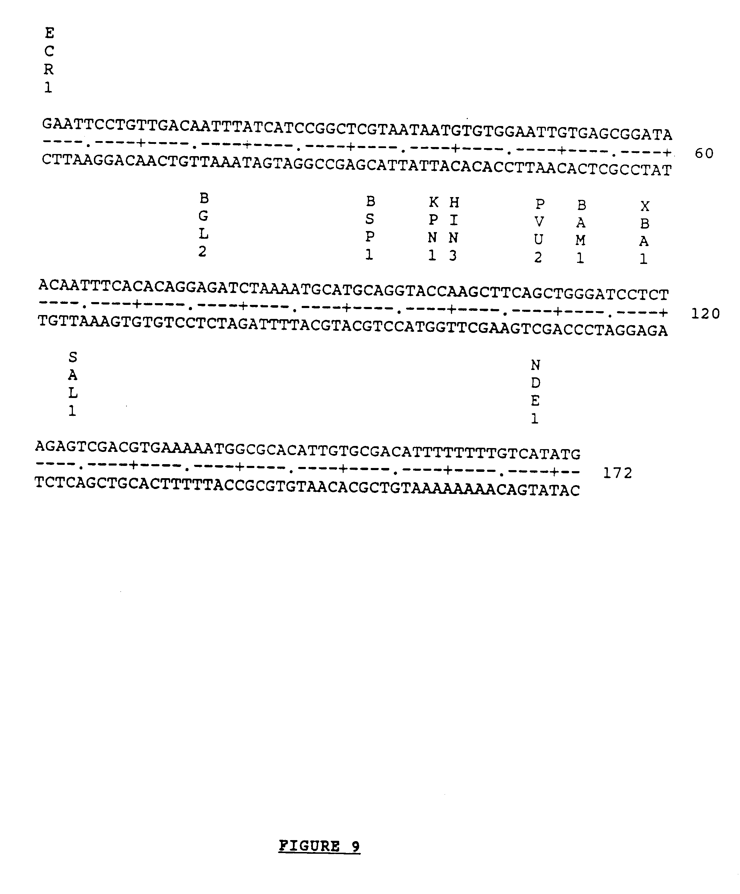

Le vecteur retenu pour exprimer la LGL dans E. coli est un plasmide dans lequel un fragment d'ADN synthétique de 172 bp contenant un promoteur de type Tac et un terminateur de transcription, a été inséré entre les sites EcoRI et NdeI de pBR322 (Bolivar, F. et al., Gene 2 (1977) 95-113). La carte de restriction du vecteur pRU276 est présentée dans la figure 8 et la séquence nucléotidique du fragment d'ADN EcoRI-NdeI dans la figure 9.
2.2. Construction du plasmide pRGLN1.3

Comme la plupart des protéines sécrétées, la LGL est synthétisée sous forme d'un précurseur composé d'un peptide signal de 22 acides aminés, situé à l'extrémité NH₂-terminale et précédant la séquence polypeptidique de la lipase mature. La détermination de la séquence de la lipase gastrique humaine (LGH) et la lipase linguale de rat (LLR) avaient montré que la LGL était plus courte de 3 acides aminés que les deux autres lipases (Moreau et al. déjà cité). Ces résultats sont présentés dans la figure 1.

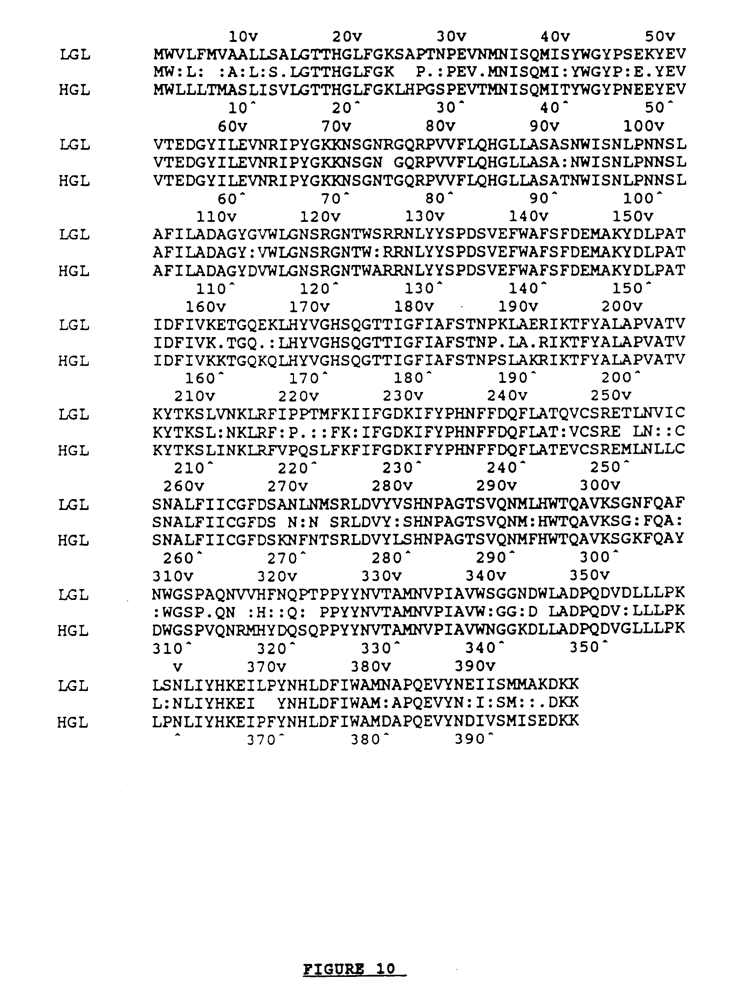

Par contre, la comparaison des séquences des précurseurs de la LGH et de la LGL ne fait apparaître aucune différence au niveau du site de coupure des séquences polypeptidiques primaires, comme cela est montré dans la figure 10. On peut donc supposer que l'absence de 3 résidus à l'extrémité NH₂-terminale de la LGL est due à l'action d'une aminopeptidase sur l'enzyme après qu'elle ait été sécrétée par la muqueuse gastrique.

De plus, il a été récemment montré que l'élimination des 4 acides aminés NH₂-terminaux de la LGH par protéolyse partielle, entraîne une perte de l'activité lipasique de l'enzyme (Bernback, S. and Blackberg, L., Biochemistry 182 (1989) 495-499).

Des constructions laissant ou non les résidus Leu-Phe-Gly à l'extrémité NH₂-terminale de la protéine synthétisée dans E. coli ont été réalisées.

Malgré les études exhaustives qui ont été faites, peu de corrélations ont pu être établies entre le niveau d'expression d'une protéine hétérologue dans une bactérie et la séquence nucléotidique de la région 5′-terminale de l'ARN messager de cette même protéine. Cependant, nombre d'observations ont permis de déduire certaines règles empiriques pouvant conduire à un niveau d'expression plus élevé dans les bactéries recombinantes.

Parmi ces "règles", on peut citer:
- la distance entre la région de Shine et Dalgarno et l'AUG initiateur comprise entre 6 et 12 nucléotides.
- une séquence de Shine et Dalgarno riche en purines (AGGA).
- une structure secondaire minimale entre Shine et Dalgarno et l'AUG initiateur.
- l'absence de structure secondaire (double brin) dans les régions de l'ARN messager contenant la séquence Shine et Dalgarno et l'AUG initiateur.

On peut tenir compte de telles contraintes dans les programmes d'analyse permettant de définir les séquences nucléotidiques de région 5′ non codantes des ARNm susceptibles de conduire aux meilleurs niveaux d'expression de protéines hétérologues particulières, telles que la LGL dans E. coli.

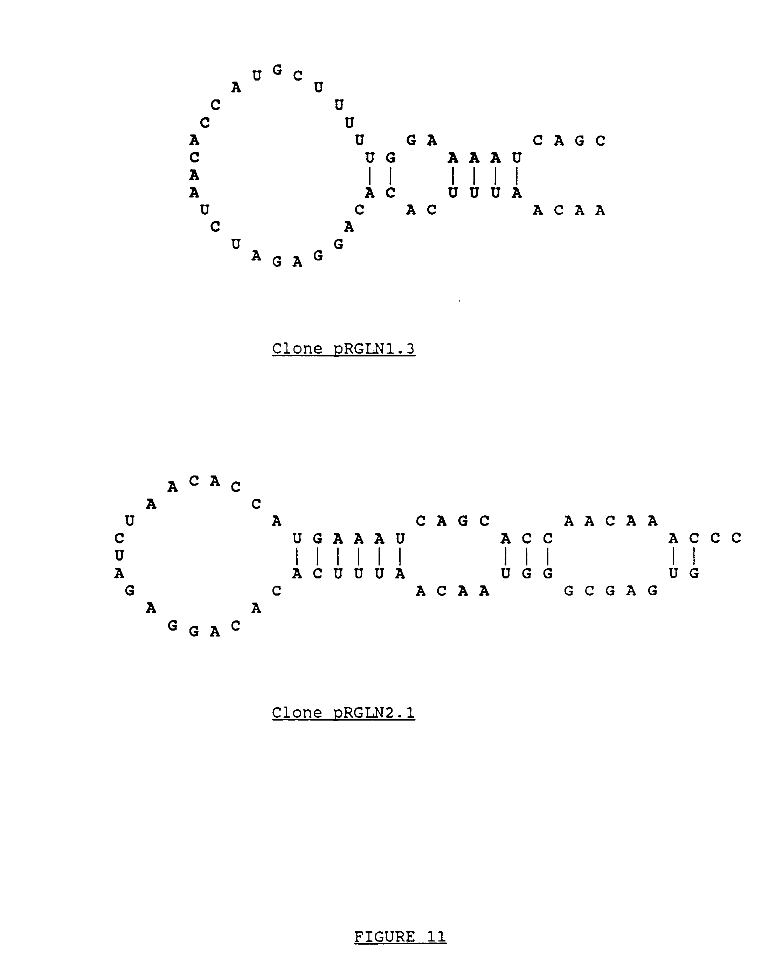

Les structures secondaires possibles autour de l'AUG initiateur des ARN messagers codant pour la LGL et issus des plasmides pRGLN1.3 et pRGLN2.1 sont présentées dans la figure 11.

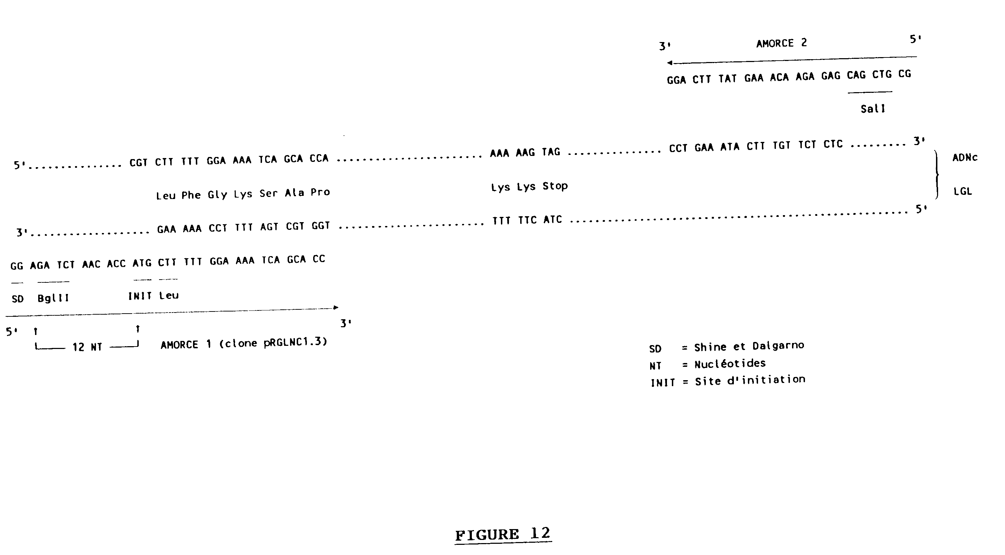

Grâce à des amorces synthétiques spécifiques présentées dans la figure 12 et par la technique d'amplification génique PCR, le cDNA codant pour la partie mature de la LGL (avec la séquence Leu-Phe-Gly) est positionné derrière un codon d'initiation de la traduction ATG et placé entre des séquences nucléotidiques telles qu'on puisse l'insérer dans le vecteur d'expression pRU276 entre les sites de restriction BglII et SalI. Du fait de la présence d'un codon ATG immédiatement en amont des séquences codant pour une LGL possédant ou non la séquence Leu-Phe-Gly, dans les constructions pRGLN.3 et pRGLN2.1, les protéines recombinantes obtenues posséderont, totalement ou partiellement, une méthionine à leur extrémité NH₂-terminale.

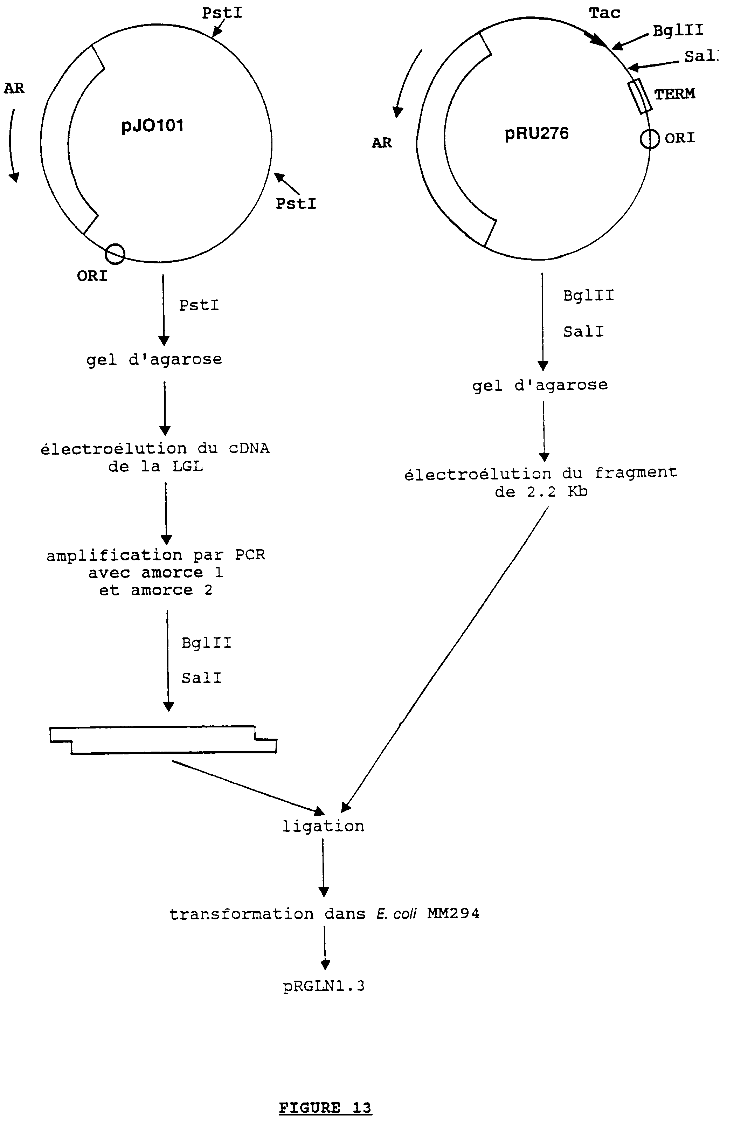

Le plasmide recombinant pRGLN1.3 dont le schéma de construction est reporté dans la figure 13 a été obtenu dans la souche E. coli MM294 puis transféré dans la souche E. coli W3110 I^{q}, fréquemment utilisée pour l'expression de protéines hétérologues. Cette souche contient le gène du répresseur LacIq situé sur un épisome non transférable F′: le répresseur synthétisé en quantité importante dans la bactérie réprime l'expression de tous les gènes placés sous contrôle d'un promoteur de type lactose.
2.3. Construction du plasmide pRGLN2.1.

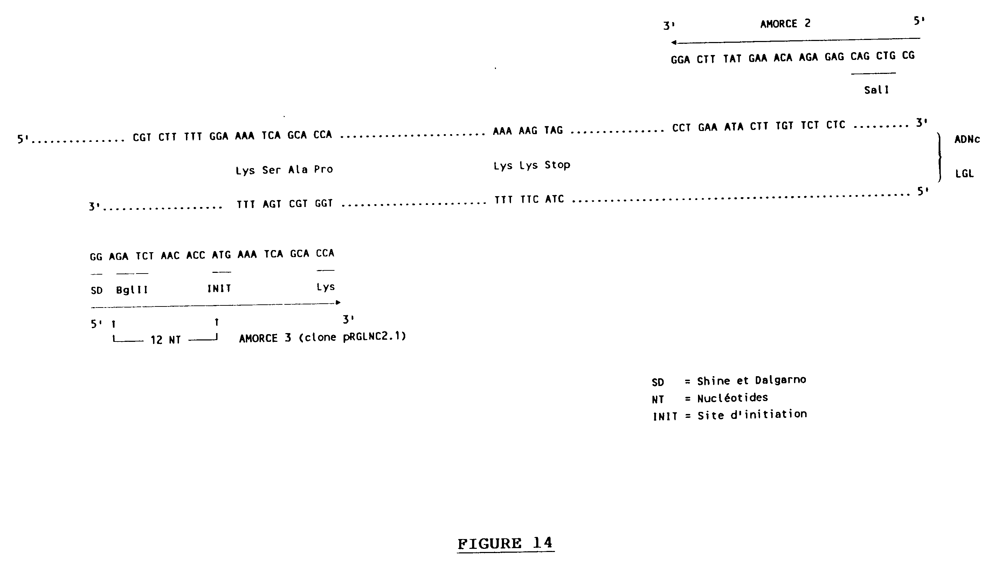

De la même façon, la plasmide pRGLN2.1 dans lequel l'ADNc de la lipase gastrique de lapin dépourvu des codons Leu-Phe-Gly de l'extrémité 5′ a été placé sous contrôle du promoteur Tac, a été construit. L'amplification génique par PCR a été réalisée grâce aux amorces présentées dans la figure 14.

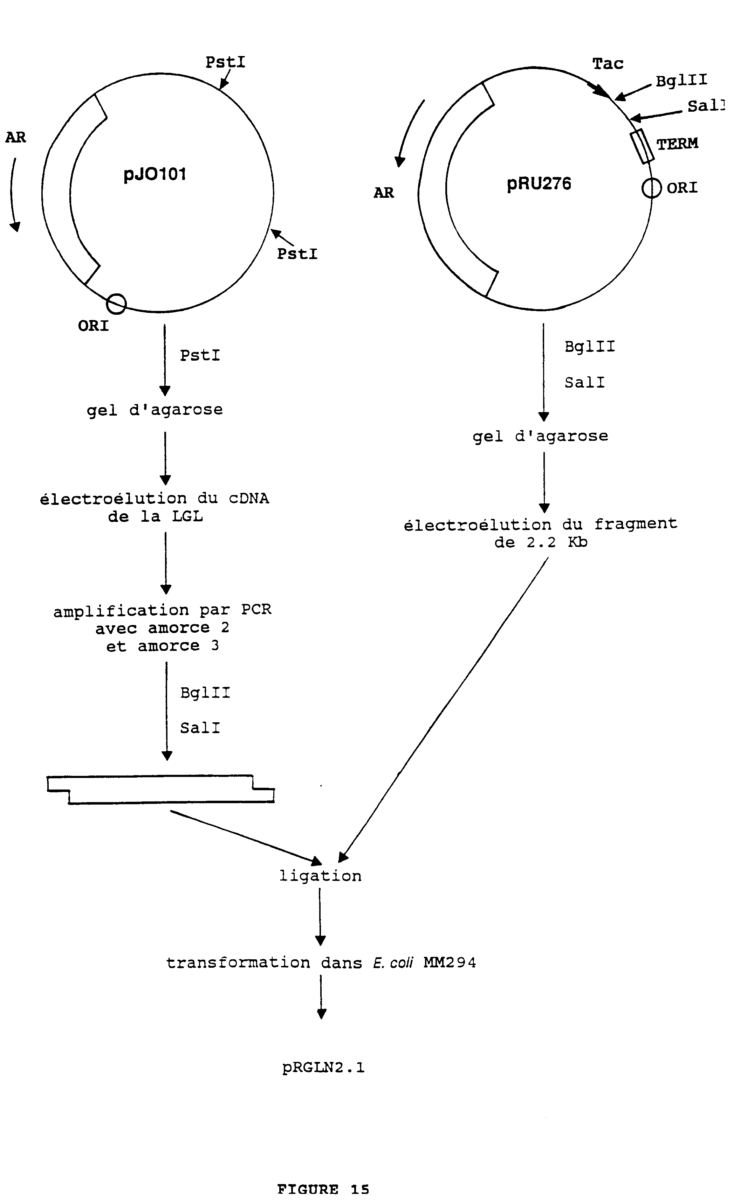

Le schéma de construction du plasmide PRGLN2.1 est présenté dans la figure 15

### Exemple n° 3 : Expression de la LGL dans E. coli.

Les plasmides pRGLN1.3 et pRGLN2.1 ont été introduits dans l'hôte E. coli W3110 I^{q}. Les bactéries transformées par les différents plasmides sont cultivées dans du milieu M9+glucose (Maniatis, T. et al., déjà cité). Pendant la phase exponentielle de croissance, l'expression de la LGL est induite par addition d'IsoPropylThioGalactopyranoside à la concentration finale de 2 mM.

Après 4 heures à 37°C, les bactéries sont récoltées, centrifugées et lavées avec du tampon PBS. Les bactéries sont ensuite lysées dans un tampon SDS/ β-mercaptoéthanol pendant 10 minutes à 100°C.

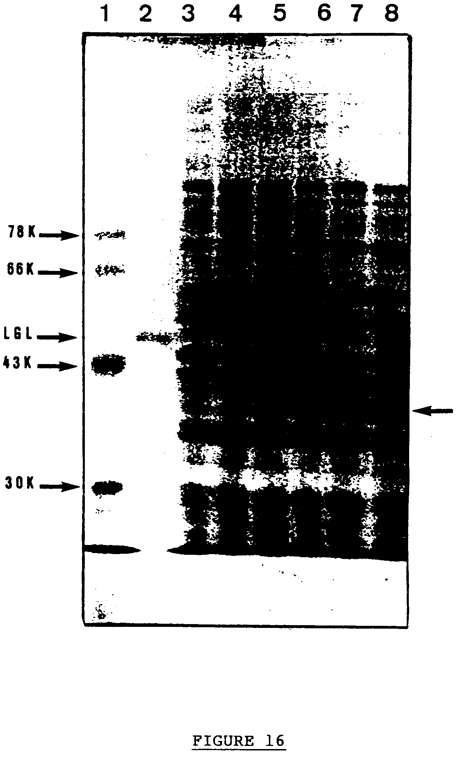

L'analyse des protéines sur gel d'électrophorèse en conditions dénaturantes permet la mise en évidence d'une bande protéique pouvant correspondre à la lipase. La protéine est exprimée à un taux tel qu'elle peut être détectée par cette technique comme ceci est montré dans la figure 16.

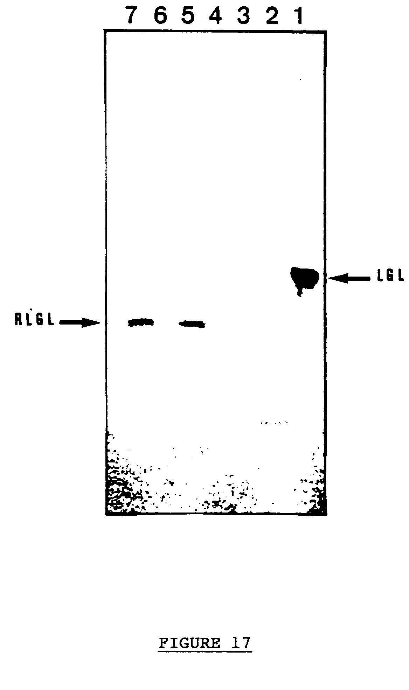

Afin de s'assurer que cette protéine, induite par l'addition d'IPTG au milieu de culture, correspond bien à la LGL, les protéines provenant de cultures de bactéries transformées par les différents plasmides induites et non-induites par l'agent chimique sont transférées sur une membrane de nylon après qu'elles aient été séparées selon leur taille par électrophorèse sur gel de polyacrylamide-SDS. Le complexe entre la LGL et l'anticorps anti-LGL peut être mis en évidence grâce à une réaction colorimétrique faisant intervenir un deuxième anticorps couplé à une enzyme, la peroxydase de raifort. Les résultats sont présentés dans la figure 17.

La détection de l'activité de la LGL recombinante, notamment celle issue d'un lysat bactérien provenant d'une culture de W3110(pRGLN1.3), est avantageusement réalisée par la méthode de Gargouri et al (gastroenterology 91 (1986) 265-275) en utilisant la tributyrine comme substrat.

## Revendications

1. Acide nucléique caractérisé en ce qu'il comprend le fragment d'ADN délimité par les nucléotides situés aux positions 120 et 1247 de l'ADN représenté sur la figure 6, ce fragment d'ADN codant pour le polypeptide délimité par les acides aminés situés aux positions 23 et 398 de la séquence en acides aminés représentée sur la figure 7, ce polypeptide correspondant à la lipase gastrique de lapin.

2. Acide nucléique selon la revendication 1, caractérisé en ce qu'il comprend en amont du fragment d'ADN représenté sur la figure 1, le fragment d'ADN délimité par les nucléotides situés aux positions 111 et 119 de l'ADN représenté sur la figure 6, et codant pour la séquence en acides aminés Leu-Phe-Gly.

3. **3.** Acide nucléique selon la revendication 1 ou la revendication 2, caractérisé en ce qu'il comprend en amont du fragment d'ADN délimité par es nucléotides situés aux positions 120 et 1247, et de celui délimité par les nucléotides situés aux positions 111 et 1247 de la figure 6, un fragment d'ADN codant pour une méthionine.

4. Acide nucléique selon la revendication 1 caractérisé en ce qu'il comprend le fragment d'ADN délimité par les nucléotides situés aux positions 54 et 1247 de l'ADN représenté sur la figure 6, ce fragment d'ADN codant pour le polypeptide de 398 acides aminés représenté sur la figure 7, ce polypeptide correspondant au précurseur de la lipase gastrique de lapin.

5. Acide nucléique caractérisé en ce qu'il comprend en amont d'un des fragments d'ADN selon l'une des revendications 1 à 4, une séquence signal codant pour un peptide signal.

6. Acide nucléique caractérisé en ce qu'il comprend l'une ou l'autre des deux séquences nucléotidiques complémentaires constituant les fragments d'ADN selon l'une des revendications 1 à 5.

7. Acide nucléique caractérisé en ce qu'il est susceptible de s'hybrider avec un acide nucléique selon la revendication 6.

8. Acide nucléique codant pour un polypeptide tel que défini dans l'une des revendications 1 à 5, et dont la séquence nucléotidique diffère, en fonction de la dégénérescence du code génétique, des séquences nucléotidiques définies dans l'une des revendications 1 à 7.

9. Acide nucléique recombinant caractérisé en ce qu'il comprend un acide nucléique selon l'une des revendications 1 à 8, inséré dans une séquence nucléotidique hétérologue vis à vis du susdit acide nucléique.

10. Acide nucléique recombinant selon la revendication 9, caractérisé en ce qu'il comprend un promoteur situé en amont de l'acide nucléique selon l'une des revendications 1 à 8, et sous le contrôle duquel la transcription dudit acide nucléique est susceptible d'être effectuée, ainsi qu'une séquence codant pour des signaux de terminaison de la transcription située en aval dudit acide nucléique.

11. Vecteur recombinant, notamment du type plasmide, cosmide ou phage, caractérisé en ce qu'il contient un acide nucléique recombinant selon la revendication 9 ou la revendication 10, en l'un de ses sites non essentiels pour sa réplication.

12. Vecteur recombinant selon la revendication 11, caractérisé en ce qu'il contient en l'un de ses sites non essentiels pour sa réplication, des éléments nécessaires pour promouvoir et contrôler l'expression d'un acide nucléique selon l'une des revendications 1 à 8, dans un hôte cellulaire, et plus particulièrement un promoteur reconnu par les polymérases de l'hôte cellulaire, notamment un promoteur inductible.

13. Hôte cellulaire, de type procaryote ou eucaryote, transformé par un vecteur recombinant selon la revendication 11 ou la revendication 12, et comprenant les éléments de régulation permettant l'expression d'un acide nucléique selon l'une des revendications 1 à 8.

14. Polypeptide caractérisé en ce qu'il est codé par un acide nucléique selon l'une des revendications 1 à 8.

15. Polypeptide selon la revendication 14, correspondant selon le code génétique universel à l'acide nucléique selon la revendication 1, et délimité par les acides aminés situés aux positions 23 et 398 de la séquence en acides aminés représentée sur la figure 7.

16. Polypeptide selon la revendication 14, correspondant selon le code génétique universel à l'acide nucléique selon la revendication 2, ce polypeptide étant constitué de la séquence en acides aminés délimitée par les acides aminés situés aux positions 23 et 398 de la figure 7, et d'une séquence Leu-Phe-Gly située en amont de cette dernière.

17. Polypeptide selon la revendication 15 ou la revendication 16, caractérisé en ce que sa séquence en acides aminés est précédée d'une méthionine.

18. Polypeptide selon la revendication 14, correspondant selon le code génétique universel à l'acide nucléique selon la revendication 4, et tel que représenté sur la figure 7.

19. Polypeptide caractérisé en ce qu'il comprend un fragment des polypeptides recombinants selon l'une des revendications 14 à 18, ou tout polypeptide modifié par substitution et/ou addition et/ou suppression d'un ou plusieurs acides aminés de ces polypeptides recombinants, ces fragments ou polypeptides modifiés conservant les propriétés enzymatiques des polypeptides recombinants susmentionnés.

20. Procédé de préparation d'un polypeptide selon l'une des revendications 14 à 19, comprenant la succession d'étapes suivantes:
- la mise en culture d'un hôte cellulaire selon la revendication 13, dans un milieu de culture approprié, et
- la récupération du polypeptide produit par ledit hôte cellulaire, soit directement à partir du susdit milieu de culture, soit après lyse de l'hôte cellulaire.

21. Composition pharmaceutique caractérisée en ce qu'elle comprend au moins un type de polypeptide selon l'une des revendications 14 à 19, le cas échéant en combinaison avec un autre polypeptide à activité lipasique, en association avec un véhicule pharmaceutiquement acceptable.

22. Utilisation d'un ou plusieurs des polypeptides selon l'une des revendications 14 à 19, pour l'obtention de médicaments destinés à faciliter l'absorption des graisses animales ou végétales ingérées par un individu sain ou atteint d'une ou plusieurs pathologies affectant ou non le taux de production de lipase gastrique.

23. Utilisation d'un ou plusieurs des polypeptides selon l'une des revendications 14 à 19, pour l'obtention de médicaments destinés au traitement des pathologies liées à l'insuffisance, voire l'absence, de production de lipase dans l'organisme, et plus particulièrement de pathologies telles que la mucoviscidose, et l'insuffisance pancréatique exocrine.

24. Utilisation d'un ou plusieurs des polypeptides selon l'une des revendications 14 à 19, ou de toute autre lipase gastrique de mammifères et dérivés, pour la mise en oeuvre de réactions de bioconversions enzymatiques (telles que hydrolyses, trans-estérifications enzymatiques), notamment sous forme immobilisée sur un support solide.
